(19) Europäisches Patentamt
European Patent Office
Office européen des brevets

(11) EP 0 946 585 B1

(12) **FASCICULE DE BREVET EUROPEEN**

(45) Date de publication et mention de la délivrance du brevet:
**17.04.2002 Bulletin 2002/16**

(21) Numéro de dépôt: **97952972.4**

(22) Date de dépôt: **22.12.1997**

(51) Int Cl.[7]: **C07J 41/00**, A61K 31/565

(86) Numéro de dépôt international:
**PCT/FR97/02379**

(87) Numéro de publication internationale:
**WO 98/28324 (02.07.1998 Gazette 1998/26)**

(54) **STEROIDES SUBSTITUES EN POSITION 11, LEUR PROCEDE DE PREPARATION, LEUR APPLICATION COMME MEDICAMENTS ET LES COMPOSITIONS PHARMACEUTIQUES LES RENFERMANT**

11-SUBSTITUIERTE STEROID VERBINDUNGEN, EIN VERFAHREN ZU IHRER HERSTELLUNG UND MEDIKAMENTEN UND PHARMAZEUTISCHE PRÄPARATE DAVON

STEROIDS SUBSTITUTED IN POSITION 11, METHOD OF PREPARATION, APPLICATION AS MEDICINES AND PHARMACEUTICAL COMPOSITIONS CONTAINING THEM

(84) Etats contractants désignés:
**AT BE CH DE DK ES FI FR GB GR IE IT LI LU NL PT SE**
Etats d'extension désignés:
**AL LT LV MK RO SI**

(30) Priorité: **23.12.1996 FR 9615829**

(43) Date de publication de la demande:
**06.10.1999 Bulletin 1999/40**

(73) Titulaire: **Aventis Pharma S.A.**
**92160 Antony (FR)**

(72) Inventeurs:
- **BOUALI, Yamina**
  **F-94800 Villejuif (FR)**
- **NIQUE, François**
  **F-94170 Le Perreux sur Marne (FR)**
- **TEUTSCH, Jean-Georges**
  **F-93500 Pantin (FR)**
- **VAN DE VELDE, Patrick**
  **F-75019 Paris (FR)**

(56) Documents cités:
**EP-A- 0 384 842** **EP-A- 0 643 071**
**FR-A- 2 640 977** **US-A- 4 859 585**

- **LU JIN ET AL: "Antiestrogenic Activity of Two 11.beta.-Estradiol Derivatives on MCF-7 Breast Cancer Cells" STEROIDS: STRUCTURE, FUNCTION, AND REGULATION., vol. 60, no. 8, août 1995, MA US, pages 512-518, XP002040770 cité dans la demande**

Remarques:
Le dossier contient des informations techniques présentées postérieurement au dépôt de la demande et ne figurant pas dans le présent fascicule.

EP 0 946 585 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne des composés stéroïdes substitués en position 11, leur procédé de préparation, leur application comme médicament et les compositions pharmaceutiques les renfermant.

L'ostéoporose est une pathologie qui se caractérise par une réduction quantitative et qualitative du tissu osseux, suffisante pour entraîner des fractures vertébrales ou périphériques, de façon spontanée ou à l'occasion de traumatismes minimes. Bien que cette affection soit d'origine multifactorielle, c'est la ménopause qui, chez la femme, constitue le facteur prépondérant de la perte osseuse ou ostéopénie.

Cette ostéopénie se manifeste par une raréfaction et une modification de l'architecture de l'os spongieux qui a pour conséquence d'accentuer la fragilité squelettique et le risque fracturaire. La perte osseuse s'accentue fortement après la ménopause en raison de la suppression de la fonction ovarienne et atteint 3 à 5 % par an pour se ralentir après 65 ans.

Dans un but thérapeutique, la carence hormonale post ménopausique peut être compensée par une hormonothérapie substitutive où l'estrogène joue un rôle majeur en préservant le capital osseux. Mais l'estrogénothérapie au long cours s'accompagne parfois d'effet indésirable sur l'appareil génital (hyperplasie endométriale, tumeur mammaire...), ce qui constitue un inconvénient majeur et limite son application.

Il convient donc de trouver d'autres composés que l'oestradiol ayant une activité estrogène dissociée, à savoir une activité estrogène au niveau osseux, tout en n'ayant pas ou peu d'activité d'hyperplasie endométriale, ni d'activité de prolifération de tumeur mammaire. La demande de brevet FR 2640977 A (29 juin 1990) décrit des stéroïdes présentant une activité antiestrogène et/ou estrogène. Ces molécules différent de celle de notre demande en ce qu'elles sont substituées par un groupement alkynyle ou non substituées en position 17 alpha. La revue Steroids (Lu jin et al. Vol 60 n°8 août 1995,512-518) divulgue également un analogue non substitué en postion 17 alpha (RU39411) qui présente une activité mixte estrogène/antiestrogène,inhibe la croissance des cellules tumorales mammaires MCF-7 et est donc utile dans le traitement du cancer du sein.

L'invention a donc pour objet les composés de formule générale (I) :

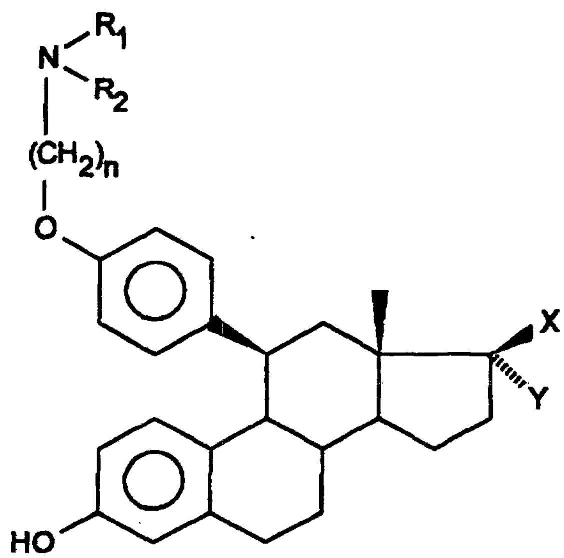

(I)

dans laquelle :

n est un entier égal à 2 ou 3,
<u>soit</u> $R_1$ et $R_2$ identiques ou différents représentent un atome d'hydrogène ou un radical alkyle renfermant de 1 à 4 atomes de carbone,
<u>soit</u> $R_1$ et $R_2$ forment ensemble avec l'atome d'azote auquel ils sont liés un hétérocycle, mono ou polyclique, saturé ou insaturé, de 5 à 15 chaînons, aromatique ou non aromatique, renfermant éventuellement de 1 à 3 hétéroatomes additionnels choisis parmi l'oxygène, le soufre et l'azote, substitué ou non substitué, X représente un radical hydroxyle éventuellement estérifié et Y représente un radical alkyle renfermant de 1 à 4 atomes de carbone, substitué ou non substitué, ainsi que leurs sels d'addition avec les acides pharmaceutiquement acceptables.

Par radical alkyle renfermant de 1 à 4 atomes de carbone, on entend les radicaux méthyle, éthyle, propyle,

isopropyle, n-butyle, isobutyle, tert-butyle.

Lorsque Y représente un radical alkyle substitué, il s'agit notamment d'un radical alkyle substitué par un ou plusieurs atomes d'halogène. A titre préféré, Y peut représenter le groupement trifluorométhyle.

Lorsque $R_1$ et $R_2$ forment ensemble avec l'atome d'azote auquel ils sont liés un hétérocycle, il s'agit notamment des hétérocycles saturés mono ou bicycliques renfermant éventuellement un autre hétéroatome choisi parmi l'oxygène et l'azote, comme des hétérocycles choisis parmi :pyrrolyle, imidazolyle, indolyle, pyridyle, pyrazinyle, pyrimidinyle, pyridazinyle, thiazolyle, oxazolyle, furazonyle, pyrazolinyle, thiazolinyle, et tout particulièrement les hétérocycles saturés suivants :

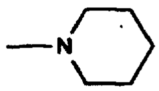 , 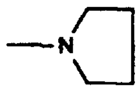 , 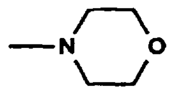 , 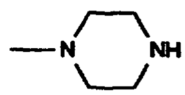 et 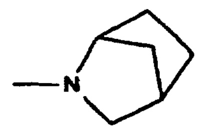

Lorsque cet hétérocycle est substitué, il l'est notamment par un groupement alkyle renfermant de 1 à 4 atomes de carbone au niveau de l'atome d'azote.

Lorsque X est un radical hydroxyle éventuellement estérifié, on entend les groupements OCO-alc1 dans lesquels alc1 est un radical alkyle renfermant de 1 à 8 atomes de carbone et de préférence les groupements -OCOMe ou OCOEt.

Par sels d'addition avec les acides pharmaceutiquement acceptables, on entend les sels d'addition formés avec des acides minéraux ou organiques sur l'amine. Il peut alors s'agir des acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

L'invention a plus particulièrement pour objet les composés de formule (I) telle que définie plus haut dans laquelle n est égal à 2, ainsi que leurs sels d'addition avec les acides pharmaceutiquement acceptables.

L'invention a plus particulièrement pour objet les composés de formule (I) telle que définie plus haut dans laquelle :n est égal à 2,
soit $R_1$ et $R_2$ identiques ou différents représentent un radical alkyle renfermant de 1 à 4 atomes de carbone,
soit $R_1$ et $R_2$ forment ensemble avec l'atome d'azote auquel ils sont liés un groupement pipéridino, pyrrolidino ou 2-azabicyclo(2.2.1)hept-2-yle,
X représente un radical hydroxyle et Y représente un radical méthyle ou éthyle.

L'invention a tout particulièrement pour objet les composés de formule (I) ainsi que leurs sels d'addition avec les acides pharmaceutiquement acceptables dont les noms suivent :

- 11β-[4-[2-(1-piperidinyl)éthoxy]phényl]-19-nor-17α-pregna-1,3,5(10)-triène-3,17β-diol,
- 17α-méthyl-11β-[4-[2-(1-piperidinyl)éthoxy]phényl]-estra-1,3,5(10)-triène-3,17β-diol,
- 17α-méthyl-11β-[4-[2-(diéthylamino)éthoxy]phényl]-estra-1,3,5(10)-3,17β-diol,
- 17α-méthyl-11β-[4-[2-(1-pyrrolidinyl)éthoxy]phényl]-estra-1,3,5(10)-triène-3,17β-diol,
- 17α-méthyl-11β-[4-[2-(2-aza-bicyclo(2.2.1)hept-2-yle) éthoxylphényl]-estra-1,3,5(10)-triène-3,17β-diol,
- 11β-[4-[2-(2-aza-bicyclo(2.2.1)hept-2-yle) éthoxy]phényl]-19-nor-17α-pregna-1,3,5(10)-triène-3,17β-diol,
- 17α-(trifluorométhyl) 11β-[4-[2-(1-pipéridinyl)éthoxy] phényl]-estra-1,3,5(10)-triène-3,17β-diol.

L'invention a également pour objet un procédé de préparation des composés de formule générale (I) telle que définie précédemment, caractérisé en ce que l'on soumet un composé de formule générale (II) :

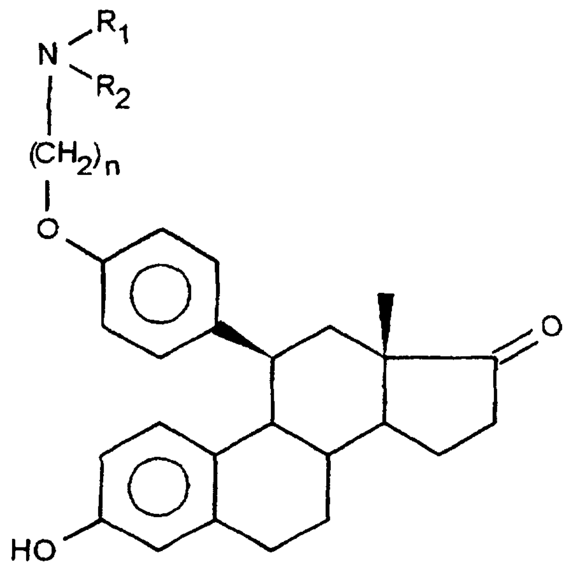

(II)

dans laquelle n, $R_1$ et $R_2$ sont tels que définis précédemment, à l'action d'un composé organométallique dérivé d'un radical alkyle renfermant de 1 à 4 atomes de carbone afin de former les composés de formule (I) dans laquelle X est un groupement hydroxyle et Y est un groupement alkyle renfermant de 1 à 4 atomes de carbone, composé de formule (I) que l'on soumet le cas échéant à une réaction d'estérification du 17-OH et/ou à une réaction de salification.

L'action d'un organométallique sur le groupement 17-céto permet d'avoir accès aux produits de formule (I) dans laquelle X est un groupement hydroxyle et Y est un groupement alkyle renfermant de 1 à 4 atomes de carbone.

L'organométallique dérivé d'un radical alkyle renfermant de 1 à 4 atomes de carbone est choisi parmi les magnésiens de formule Y-MgHal et les lithiens de formule Y-Li dans lesquelles Y est tel que défini précédemment et Hal représente un atome d'halogène. De préférence la réaction a lieu en présence du chlorure de cérium. Dans un mode préféré d'exécution du procédé, Hal représente un atome de chlore, de brome ou d'iode, de préférence de brome.

Pour obtenir des composés de formule (I) dans laquelle X est un radical hydroxyle et Y un groupement $CF_3$, la réaction s'effectue par action de $CF_3SiMe_3$ sur le 17-céto suivi de l'action d'un réactif de déprotection tel que le fluorure de tétrabutylammonium.

L'invention a également pour objet un procédé de préparation des composés de formule générale (I) telle que définie précédemment, avec Y représentant un radical alkyle renfermant de 2 à 4 atomes de carbone, caractérisé en ce que l'on soumet un composé de formule générale (III) :

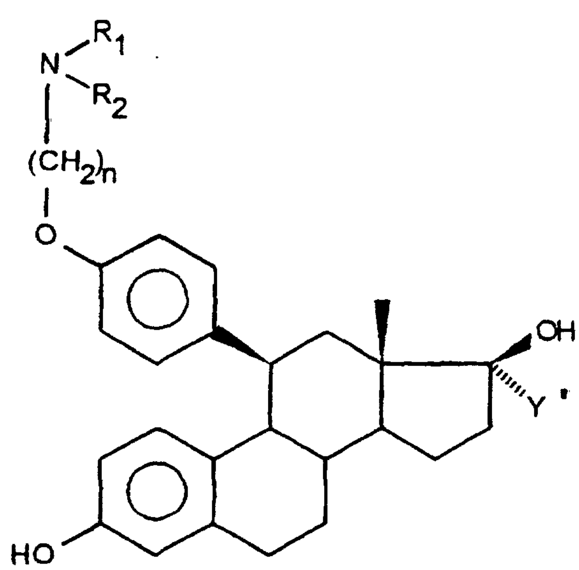

(III)

dans laquelle n, $R_1$ et $R_2$ sont tel que définis précédemment et dans laquelle Y' représente un groupement alkényle ou alkynyle renfermant de 2 à 4 atomes de carbone, à l'action d'un agent de réduction de la double liaison ou de la triple liaison, afin d'obtenir les composés de formule (I) dans laquelle X est un groupement hydroxyle et Y est un groupement alkyle renfermant de 1 à 4 atomes de carbone, composé de formule. (I) que l'on soumet le cas échéant à une réaction d'estérification du 17-OH et/ou à une réaction de salification.

La réaction de réduction totale peut s'effectuer par action d'hydrogène en présence d'un catalyseur tel que le palladium sur charbon ou un catalyseur au rhodium tel que le réactif de Wilkinson.

Les réactions d'estérification et de salification sont effectuées par les méthodes courantes connues de l'homme du métier.

Les composés de formule générale (I) ainsi que leurs sels d'addition avec les acides pharmaceutiquement acceptables possèdent des activités estrogène, anti-estrogène et anti-prolifératives.

A ce titre, les composés de formule (I) peuvent être utilisés, dans le traitement des troubles liés à une hypofolliculinie, par exemple, les aménorrhées, les dysménorrhées, les avortements répétés, les troubles prémenstruels, dans le traitement de certaines pathologies estrogéno-dépendantes telles que les adénomes ou carcinomes prostatiques, les carcinomes mammaires et ses métastases ou dans le traitement des tumeurs bénignes du sein, en tant qu'anti-utérotrophique ainsi que dans le traitement substitutif de la ménopause ou de la périménopause.

Parmi les symptômes et les conséquences liées à la ménopause on entend plus précisément les bouffées de chaleur, les sueurs, l'atrophie et la sécheresse vaginale, les symptômes urinaires et à long terme la diminution de la masse osseuse et l'augmentation du risque de fracture, ainsi que la perte de la protection cardio-vasculaire offerte par les estrogènes.

En particulier, les composés de formule (I) ainsi que leurs sels d'addition avec les acides pharmaceutiquement acceptables, peuvent ainsi être utilisés dans la prévention ou le traitement de l'ostéoporose.

Les composés de formule (I) ainsi que leurs sels d'addition avec les acides pharmaceutiques acceptables, peuvent également être utilisés dans la prévention ou le traitement de l'ostéoporose chez l'homme.

Ils peuvent également être utilisés dans la prévention ou le traitement des ostéoporoses secondaires (par exemple cortisoniques, liées à une immobilisation).

Les composés de formule (I) ainsi que leurs sels d'addition avec les acides pharmaceutiquement acceptables, possèdent notamment une activité estrogénique dissociée.

Par activité estrogénique dissociée, on entend une activité estrogénique au niveau osseux tout en ne manifestant qu'une activité minimale au niveau utérin, n'entraînant ainsi pas de prolifération endométriale (activité bien inférieure à celle de l'oestradiol).

Par ailleurs, les composés selon l'invention présentent les avantages suivants :

- Ils présentent une activité antiestrogène au niveau du sein. A l'opposé de l'oestradiol ils ne stimulent pas la croissance de cellules tumorales mammaires humaines et même peuvent inhiber leur croissance. Les composés selon l'invention sont donc particulièrement avantageux pour le traitement de la ménopause chez les femmes à risque de cancer mammaire (antécédents familiaux) qui sont donc exclues d'un traitement substitutif par l'oestradiol. Ils peuvent être également utilisables dans le traitement des cancers mammaires.
- Ils entraînent un abaissement du taux de cholestérol sérique à un niveau équivalent à celui induit par l'oestradiol. Ils renforcent ainsi la protection cardio-vasculaire.
- Enfin, les composés selon l'invention ne présentant pas d'activité estrogène au niveau utérin, ne nécessitent pas d'être administrés en association avec un composé progestomimétique.

L'invention a donc pour objet les composés de formule générale (I), ainsi que leurs sels d'addition avec les acides pharmaceutiquement acceptables, à titre de médicaments.

L'invention a plus particulièrement pour objet les composés de formule (I) ainsi que leurs sels d'addition avec les acides pharmaceutiquement acceptables, à titre de médicaments destinés à la prévention ou au traitement de l'ostéoporose.

L'invention a tout particulièrement pour objet les composés de formule générale (I), ainsi que leurs sels d'addition avec les acides pharmaceutiquement acceptables, à titre de médicament destiné à la prévention ou au traitement de l'ostéoporose, ne présentant que peu ou pas d'activité estrogène au niveau utérin.

Enfin l'invention a tout particulièrement pour objet les composés de formule générale (I), ainsi que leurs sels d'addition avec les acides pharmaceutiquement acceptables, à titre de médicament destiné à la prévention ou au traitement de l'ostéoporose, des femmes à risque de tumeurs mammaires.

L'invention s'étend aux compositions pharmaceutiques renfermant comme principe actif au moins l'un des médicaments tels que définis ci-dessus.

Les composés de formule (I) ainsi que leurs sels d'addition avec les acides pharmaceutiquement acceptables, sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparations injectables, de pommades, de crèmes, de gels, de microsphères, d'implants, d'anneaux intravaginal, de patchs, de sprays, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie utile varie en fonction de l'affection à traiter et de la voie d'administration ; elle peut varier par exemple de 0,5 à 100 mg par jour chez l'adulte par voie orale.

Les composés de formule générale (II) et (III) sont des composés connus et décrits dans les brevets suivants : EP-B-0097572, FR-B-2640977, EP-B-305942.

Les exemples ci-dessous illustrent l'invention sans toutefois la limiter.

**EXEMPLE 1 : 17α-méthyl-11β-[4-[2-(1-piperidinyl) éthoxy] phényl]-estra-1,3,5(10)-triène-3,17β-diol.**

Après déshydratation de 1,06 g de $CeCl_3$(III), $7H_2O$ sous pression réduite à 140°C on ajoute sous atmosphère inerte et à température ambiante 10,6 ml de tétrahydrofuranne (THF) puis, après agitation pendant 2 heures on ajoute à -70°C, 1,89 ml de solution éthérée de méthyllithium 1,6M et agite 30 minutes à -75°C. On ajoute ensuite à cette suspension 268 mg de 3-hydroxy-11β-[4-[2(1-piperidinyl)éthoxy]phényl]-estra-1,3,5(10)-triène-17-one en solution dans 3 ml de THF/ siliporite et agite à cette température pendant 1 heure. Après avoir ajouté 15 ml d'une solution saturée de chlorure d'ammonium et 20 ml d'acétate d'éthyle, on filtre, lave, sèche et évapore sous pression réduite afin d'obtenir 277 mg de produit brut attendu. Ce produit est purifié par chromatographie sur colonne de silice en éluant avec le mélange chlorure de méthylène 90/méthanol 10/hydroxyde d'ammonium 0,5. On obtient 232 mg de produit que l'on recristallise dans le mélange dichloro méthane/éther isopropylique et on obtient 180 mg de produit pur attendu.
F = 155°C
IR ($CHCl_3$)

| | |
|---|---|
| -OH | 3602 $cm^{-1}$ + absorption générale |
| aromatique | 1610 $cm^{-1}$, 1580 $cm^{-1}$, 1512 $cm^{-1}$ |

RMN ($CDCl_3$)

| | |
|---|---|
| 0,51 (s) | Me 18 |
| 1,29 (s) | Me en 17 |
| 3,98 (m) | O-C<u>H</u>$_2$-CH$_2$-N, C<u>H</u>-Ph ($H_{11}$) |
| 6,41 | $H_2$, $H_4$ cycle A, $H'_3$, $H'_5$ du phényl en 11 |
| 6,78 (d) | $H_1$ du cycle A |
| 6,94 | $H'_2$, $H'_6$ du phényl en 11 |

**EXEMPLE 2 : 11β-[4- [2-(1-piperidinyl)éthoxy]phényl]-19-nor-17α-pregna-1,3,5(10)-triène-3,17β-diol.**

A une solution sous atmosphère inerte de 192 mg de 11β-[4-[2-(1-piperidinyl)éthoxy]phényl]-19-nor-17α-pregna-1,3,5(10)-trièn-20-yne-3,17β-diol dans 6 ml d'éthanol on ajoute 20 mg de palladium sur charbon actif (9,5 %) et agite sous pression de 1660 mbar d'hydrogène pendant 1 heure 45 minutes. On filtre la suspension et évapore sous pression réduite. On obtient 193 mg de produit brut que l'on purifie par chromatographie sur colonne de silice greffée (Lichrosorb RP18) en éluant avec le mélange méthanol 90/eau 10. On obtient 137 mg de produit que l'on recristallise dans le mélange dichlorométhane/éther isopropylique et on obtient 114 mg de produit pur attendu. F = 231°C
IR ($CHCl_3$)

| | |
|---|---|
| -OH | 3600 $cm^{-1}$ + absorption générale |
| aromatique | 1610 $cm^{-1}$, 1581 $cm^{-1}$, 1512 $cm^{-1}$ |

RMN ($CDCl_3$ + 2 gouttes de $C_5D_5N$)

| | |
|---|---|
| 0,47 (s) | Me 18 |
| 1,01 (t) | CH$_2$-C<u>H</u>$_3$ |

(suite)

| | |
|---|---|
| 2,47 | -CH_2-N-CH_2- (pipéridine) |
| 2,71 | $O-CH_2-CH_2-N$ |
| 3,99 (m) | $O-CH_2-CH_2-N$, CH-Ph ($H_{11}$) |
| 6,48 (dd) | $H_2$ |
| 6,59 | $H'_3$, $H'_5$ (phényl en 11) |
| 6,63 (d) | $H_4$ (cycle A), |
| 6,80 (d) | $H_1$ (cycle A) |
| 6,96 | $H'_2$, $H'_6$ du phényl en 11 |
| 9,94 | 3-OH |

**EXEMPLE 3 : 11β-[4-[2-[2-azabicyclo (2.2.1) hept-2-yl] éthoxy] phényl] 17α-méthyl-estra-1,3,5(10)-trièn-3-17β-diol.**

On opère comme à l'exemple 1 en utilisant au départ 3,70 g de $CeCl_3$, $7H_2O$ et 37 ml de tétrahydrofuranne et 6,7 ml de solution éthérée de méthyllithium (1,6M). A la suspension obtenue refroidie à -78°C, on ajoute lentement 966 mg de 11β-[4-[2-[2-azabicyclo (2.2.1.) hept-2-yl] éthoxy] phényl] 3-hydroxy estra-1,3,5(10)-trièn-17-one en solution dans 8 ml de tétrahydrofuranne, agite 45 minutes et poursuit la synthèse comme à l'exemple 1. On obtient 874 mg de produit brut. Après chromatographie sur silice (éluant : $CH_2Cl_2$ - $CH_3OH$ - $NH_4OH$ 90-10-0,7 puis AcOEt-TEA 88-12), on obtient 442 mg de produit attendu. F = 163-164°C.
IR ($CHCl_3$)

| | |
|---|---|
| -OH | 3602 $cm^{-1}$ + absorption générale |
| aromatique | 1610 $cm^{-1}$, 1581 $cm^{-1}$, 1512 $cm^{-1}$ |

RMN ($CDCl_3$)

| | |
|---|---|
| 0,51 (s) | Me en 18 |
| 1,29 (s) | Me en 17 |
| 3,85 à 4,05 | $O-CH_2-CH_2-N$, CH-Ph ($H_{11}$) |
| 6,41 | $H_2$, $H_4$ cycle A, |
| 6,77 (d) | $H_1$ du cycle A |
| 6,46-6,95 | H du phényl en 11 |

**Préparation du 11β-[4-[2-[2-azabicyclo (2.2.1.) hept-2-yl] éthoxy] phényl] 3-hydroxy estra-1,3,5(10)-trièn-17-one utilisé au départ de l'exemple 3.**

On mélange 1,1 g de 3-hydroxy 11β-[4-(iodoéthoxy) phényl] estra-1,3,5(10)-trièn-17-one en solution dans 20 ml de tétrahydrofuranne et 1,03 g de 2-azabicyclo [2.2.1.] heptane et agite 1 heure et demie à la température du reflux sous atmosphère d'azote. On évapore le tétrahydrofuranne, reprend le résidu dans l'acétate d'éthyle, ajoute de l'eau, extrait à l'acétate d'échyle, sèche, évapore le solvant et obtient après chromatographie sur silice (éluant $CH_2Cl_2$ - $CH_3OH$ - $NH_4OH$ 90-10-0,5) 0,97 g de produit attendu.
Rf = 0,27.

**EXEMPLE 4 : 17α-méthyl 11β-[4-[2-(1-pyrrolidinyl) éthoxy] phényl] estra-1,3,5(10)-trièn-3,17β-diol.**

On opère comme à l'exemple 1 en utilisant au départ 3,24 g de $CeCl_3$, $7H_2O$, 30 ml de tétrahydrofuranne, 5,85 ml de méthyllithium puis 850 mg de 3-hydroxy 11β-[4-[2-(1-pyrrolidinyl) éthoxy] phényl] estra-1,3,5(10)-trièn-17-one en solution dans 8,5 ml de tétrahydrofuranne. Après chromatographie sur silice (éluant : $CH_2Cl_2$ - $CH_3OH$ - $NH_4OH$ 92-8-0,5), on obtient 615 mg de produit attendu. F = 155-157°C.
IR ($CHCl_3$)

| | |
|---|---|
| -OH | 3603 $cm^{-1}$ + absorption générale |
| aromatique | 1610 $cm^{-1}$, 1581 $cm^{-1}$, 1512 $cm^{-1}$ |

RMN ($CDCl_3$)

| | |
|---|---|
| 0,51 (s)<br>1,29 (s)<br>3,99<br>6,38 (dd)<br>6,40 (d)<br>6,77 (d)<br>6,49-6,95 | Me en 18<br>Me en 17<br>O-C<u>H</u>$_2$-$CH_2$-N, C<u>H</u>-Ph ($H_{11}$)<br>$H_2$ cycle A<br>$H_4$ cycle A,<br>$H_1$ cycle A<br>H du phényl en 11 |

**Préparation du 3-hydroxy 11β-[4-[2-(1-pyrrolidinyl) éthoxy] phényl] estra-1,3,5(10)-trièn-17-one utilisé au départ de l'exemple 4.**

On opère comme à la préparation du produit de départ de l'exemple 3 en utilisant 1,1 g de dérivé stéroïde iodé dans 20 ml de tétrahydrofuranne et 1 ml de pyrrolidine. On obtient 864 mg de produit attendu après chromatographie sur silice (éluant : $CH_2Cl_2$ - $CH_3OH$ - $NH_4OH$ 92-8-0,2). Rf = 0,29.

**<u>EXEMPLE 5</u> : 11β-[4- [2-(diéthylamino) éthoxy] phényl] 17α-méthyl estra-1,3,5(10)-trièn-3,17β-diol.**

On opère comme à l'exemple 1 en utilisant au départ 3,62 g de chlorure de $CeCl_3$, $7H_2O$, 36 ml de tétrahydrofuranne et 6,5 ml de méthyllithium dans l'éther (1,6M) puis 898 mg de 3-hydroxy 11β-[4-[2-(diéthylamino) éthoxy] phényl] estra-1,3,5(10)-trièn-17-one en solution dans 9 ml de tétrahydrofuranne. Après chromatographie sur silice (éluant : $CH_2Cl_2$-$CH_3OH$ - $NH_4OH$ 92-8-0,5), on obtient 686 mg de produit attendu. F = 159-160°C.
IR ($CHCl_3$)

| | |
|---|---|
| -OH | 3602cm$^{-1}$ + absorption générale |
| aromatique | 1610cm$^{-1}$, 1581cm$^{-1}$, 1512cm$^{-1}$ (F), 1500cm-1 (ep) |

RMN ($CDCl_3$)

| | |
|---|---|
| 0,47 (s)<br>1,05 (t)<br>1,28 (s)<br>2,65 (m)<br>3,95 (t)<br>6,31 (d)<br>6,38 (dd)<br>6,80 (d)<br>6,56 et 6,93 | Me 18<br>-N-($CH_2$-C<u>H</u>$_3$)$_2$<br>Me en 18<br>-N-(C<u>H</u>$_2$-C<u>H</u>$_3$)$_2$<br>O-C<u>H</u>$_2$-$CH_2$-N,<br>$H_4$ (cycle A)<br>$H_2$ (cycle A)<br>$H_1$ (cycle A)<br>H du phényl en 11 |

**Préparation du 3-hydroxy 11β-[4-[2-(diéthylamino) éthoxy] phényl] estra-1,3,5(10)-trièn-17-one.**

On opère comme à la préparation du produit de départ de l'exemple 3 en utilisant 1,1 g de dérivé stéroide iodé dans 20 ml de tétrahydrofuranne et 2 ml de diéthylamine. On obtient 898 mg de produit attendu après chromatographie sur silice (éluant : $CH_2Cl_2$ - $CH_3OH$ - $NH_4OH$ 92-8-0,2). Rf = 0,24.

**<u>EXEMPLE 6</u> : 17α-(trifluorométhyl) 11β-[4-[3-(1-pipéridinyl) propyl] phényl] estra-1,3,5(10)-trièn-3,17β-diol.**

On chauffe 2 heures à 120°C sous $10^{-2}$ mbar 83 mg de fluorure de tétrabutylammonium ($Me_4NH,4H_2O$) puis laisse revenir à température ambiante sous atmosphère inerte. On ajoute 237 mg de 3-hydroxy 11β-[4-[2-(1-pipéridinyl) éthoxy] phényl] estra-1,3,5(10)-trièn-17-one en solution dans 3 ml de tétrahydrofuranne, refroidit à +4°C et ajoute 0,3 ml de triméthyl (trifluorométhyl)-silane puis agite 2 heures à cette température. On ajoute 4 ml de fluorure de tétrabutylammonium en solution dans le tétrahydrofuranne, agite 3 heures et demie à température ambiante, ajoute de l'eau, extrait au chlorure de méthylène,lave à l'eau, sèche et évapore les solvants sous pression réduite. On chromatographie le résidu sur silice (éluant : $CH_2Cl_2$ - MeOH - $NH_4OH$ 90-10-0,1) et obtient 127 mg de produit attendu.

IR ($CHCl_3$)

| | |
|---|---|
| -OH | 3598 $cm^{-1}$ + absorption générale |
| aromatique | 1610 $cm^{-1}$, 1580 $cm^{-1}$, 1512 $cm^{-1}$ |

RMN ($CDCl_3$)

| | |
|---|---|
| 0,56 (s) | Me 18 |
| 4,00 (m) | O-C$\underline{H}_2$-C$\underline{H}_2$-N, C$\underline{H}$-Ph ($H_{11}$) |
| 6,37 (dd) | $H_2$ (cycle A) |
| 6,41-6,93 | H'2, $H'_3$ (phényl en 11) |
| 6,41 (d) | $H_4$ (cycle A) |
| 6,77 (d) | $H_1$ (cycle A) |

**TESTS PHARMACOLOGIQUES**

1) Effet sur la prolifération de cellules mammaires

L'activité proliférative des molécules est étudiée comparativement à celle de l'oestradiol sur les cellules mammaires humaines MCF-7 en culture.

Pour mettre en évidence un effet agoniste de l'oestradiol et/ou des molécules testées, le milieu de culture d'entretien des cellules (riche en facteurs de croissance et en stéroïdes) est remplacé par un milieu appauvri, entre autres dépourvu de stéroides (DMEM supplémenté par 5 % de sérum déstéroïdé et sans rouge de phénol). Les cellules subissent ce sevrage deux jours avant le début de l'essai.

Après 7 jours de culture en présence des produits à étudier, la prolifération cellulaire est évaluée par dosage du DNA. Dans chaque essai, l'effet de l'oestradiol à $10^{-10}$M (croissance cellulaire en présence d'oestradiol moins croissance cellulaire en présence du solvant) détermine le 100 % de l'activité agoniste. L'activité des molécules est évaluée en comparaison à ce témoin interne. Les molécules induisant une croissance cellulaire identique à celle observée avec le solvant seul sont classées "inactives", celles induisant une croissance cellulaire inférieure à celle observée avec le solvant sont classées "inhibiteur".

| | E2 | Ex.2 | Ex.3 |
|---|---|---|---|
| Activité | Agoniste | Inactif | Inhibiteur |

2) Les composés selon l'invention sont testés afin de déterminer leur effet sur la masse osseuse et sur l'activité de formation et de résorption dans le modèle de la rate ovariectomisée à l'âge de 3 mois. Les animaux sont traités en préventif.

**Animaux :**

| | |
|---|---|
| Espèce | rat |
| Souche | Sprague-Dawley |
| Sexe | femelle |
| Poids | 250 g à 280 g |

Nbre d'animaux/groupe 8

**Produits :**

1 - Produit à tester : Produit de l'exemple 1.

* véhicule (s) : huile de maïs, méthylcellulose 0,5 %
* dose(s) : une dose par produit testé (0,3 mg/kg/j)
* nombre d'administrations : une fois/jour ; 5 jours/semaine pendant 4 semaines
* voie d'administration : voie orale pour les produits
* volumes : 5 ml/kg (p.o.)
* délai entre la dernière injection et le sacrifice : 24 heures
* nombre d'administrations : 20.

2 - Produit de référence : le 17β oestradiol est administré par voie sous cutanée à la dose 0,1 mg/kg/j en solution dans un mélange d'huile de germe de maïs-alcool benzylique (99:1, v/v) sous un volume de 0,2 ml/kg.

**Protocole expérimental**

Animaux

L'étude est réalisée chez des rats femelles ovariectomisées à l'âge de 3 mois. Les animaux sont maintenus dans une pièce climatisée (température 20°C ± 2°C) et groupés par 4 dans des boîtes. Les animaux reçoivent, ad libitum, de l'eau déminéralisée et des aliments comprimés (bouchons : AO4CR-10 UAR).

Chirurgie

Des rats femelles âgées de 3 mois pesant environ 250 g sont ovariectomisées sous anesthésie à l'Imalgène 1000, à la dose de 100 mg/kg par voie intrapéritonéale (i.p.) et sous un volume de 1 ml/kg. Ils reçoivent également du Nembutal (3 mg/kg i.p. sous un volume de 0,3 ml/kg).

Après incision latérale, les plans cutanés et musculaires sont sectionnés. L'exérèse de chaque ovaire se fait après ligature de l'oviducte.

Les rats témoins "SHAM" sont anesthésiés dans les mêmes conditions. Après incision des plans cutanés et musculaires, chaque ovaire est exposé puis replacé in situ.

**Traitement**

Les effets des produits sont déterminés en traitement préventif. Ils sont administrés immédiatement après l'ovariectomie. Les animaux répartis en groupes de 8.
Groupe 1 : rats témoins "SHAM" recevant le ou les véhicules
Groupe 2 : rats témoins "OVX" recevant le ou les véhicules
Groupes X : rats "OVX" recevant respectivement les doses définies du ou des produits à tester.

Prélèvements sanguins

Au terme des 4 semaines (durée de l'étude) les animaux sont décapités par guillotine. Les sérums recueillis après centrifugation sont conservés à -20°C.

Un bilan lipidique sera établi à partir des dosages sériques du cholestérol total, des triglycérides et des phospholipides sur une aliquote de sérum de 500 µl. La baisse du taux de cholestérol sérique est exprimée en % par rapport au taux présenté par les animaux ovariectomisés ne recevant que le solvant.

Prélèvements d'organes

Après sacrifice des animaux, les organes suivants sont prélevés :

- tractus génital
  Les utérus sont prélevés. Ces derniers sont pesés. L'augmentation du poids est exprimée, en % du poids de l'utérus des animaux ovariectomisés ne recevant que le solvant.
- au niveau osseux :
  La masse osseuse (BMD ou Bone mineral density = densité minérale osseuse) est mesurée par absorptiométrie biphotonique à rayons X en double énergie (DEXA). Les mesures sont réalisées sur les os excisés et débarrassés de tous les tissus mous. La BMD (Bone mineral density) est mesurée sur l'os entier ainsi que sur la partie métaphysaire au niveau de l'extrémité proximale pour le tibia gauche. Cette zone est définie comme étant la région la plus riche en os trabéculaire ; et par conséquent, est la plus sensible aux variations de volume osseux

et de densité minérale osseuse.

Les résultats sont exprimés en % selon la formule :

$$\frac{\text{BMD produit testé - BMD OVX}}{\text{BMD SHAM - BMD OVX}} \times 100$$

| | Dose | OS TIBIA | UTERUS | Cholest. |
|---|---|---|---|---|
| | mg/kg | densité % | Poids % W | % |
| E2 | 0,1 sc | 105 | 359 | - 35 |
| Ex.1 | 0,3 po | 75 | 76 | - 43 |
| Ex.3 | 0,3 po | 46 | 37 | - 40 |
| OVX | | 0 | | |
| SHAM | | 100 | | |

**<u>Conclusions</u> :**

Les composés selon l'invention offre une protection osseuse efficace (= 75 %), tout en montrant une activité utérotrophique minimale en comparaison de celle provoquée par l'oestradiol. De plus, on observe une baisse significative du taux de cholestérol total.

## Revendications

**1.** Les composés de formule générale (I) :

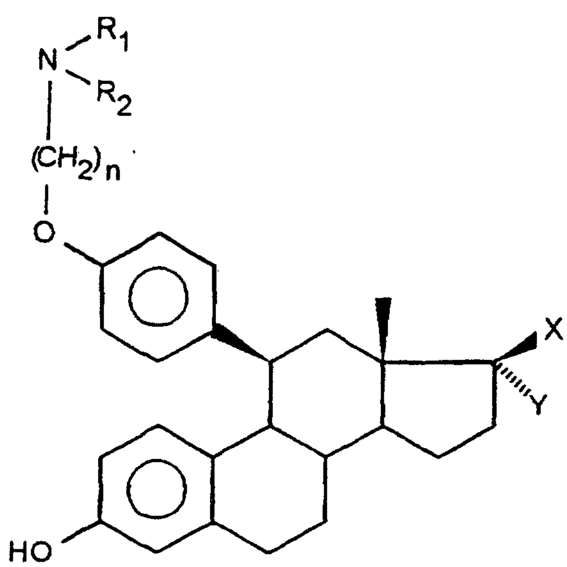

(I)

dans laquelle :

n est un entier égal à 2 ou 3,
<u>soit</u> $R_1$ et $R_2$ identiques ou différents représentent un atome d'hydrogène ou un radical alkyle renfermant de 1 à 4 atomes de carbone,
<u>soit</u> $R_1$ et $R_2$ forment ensemble avec l'atome d'azote auquel ils sont liés un hétérocycle, mono ou polyclique, saturé ou insaturé, aromatique ou non aromatique, de 5 à 15 chaînons, renfermant éventuellement de 1 à 3

hétéroatomes additionnels choisis parmi l'oxygène, le soufre et l'azote, substitué ou non substitué,
X représente un radical hydroxyle éventuellement estérifié et
Y représente un radical alkyle renfermant de 1 à 4 atomes de carbone, substitué ou non substitué, ainsi que leurs sels d'addition avec les acides pharmaceutiquement acceptables.

**2.** Composés de formule générale (I) telle que définie à la revendication 1 dans laquelle n est égal à 2, ainsi que leurs sels d'addition avec les acides pharmaceutiquement acceptables.

**3.** Composés de formule générale (I) telle que définie à la revendication 1 ou 2 dans laquelle n est égal à 2, soit $R_1$ et $R_2$ identiques ou différents représentent un radical alkyle renfermant de 1 à 4 atomes de carbone,
soit $R_1$ et $R_2$ forment ensemble avec l'atome d'azote auquel ils sont liés un groupement pipéridino, pyrrolidino ou 2-aza-bicyclo(2.2.1)hept-2-yle,
X représente un radical hydroxyle et Y représente un radical méthyle ou éthyle, ainsi que leurs sels d'addition avec les acides pharmaceutiquement acceptables.

**4.** Composés de formule générale (I) telle que définies à l'une quelconque des revendications 1 à 3 ainsi que leurs sels d'addition avec les acides pharmaceutiquement acceptables dont les noms suivent :

- 11β-[4- [2- (1-piperidinyl)éthoxy]phényl]-19-nor-17α-pregna-1,3,5(10)-triène-3,17β-diol,
- 17α-méthyl-11β-[4-[2-(1-piperidinyl)éthoxy]phényl]-estra-1,3,5(10)-triène-3,17β-diol,
- 17α-méthyl-11β-[4-[2-(diéthylamino)éthoxy]phényl]-estra-1,3,5(10)-3,17β-diol,
- 17α-méthyl-11β-[4-[2-(1-pyrrolidinyl)éthoxy]phényl]-estra-1,3,5(10)-triène-3,17β-diol,
- 17α-méthyl-11β-[4-[2-(2-aza-bicyclo(2.2.1)hept-2-yle) éthoxy]phényl]-estra-1,3,5(10)-triène-3,17β-diol,
- 11β-[4-[2-(2-aza-bicyclo(2.2.1)hept-2-yle) éthoxy]phényl]-19-nor-17α-pregna-1,3,5(10)-triène-3,17β-diol,
- 17α-(trifluorométhyl) 11β-[4- [3- (1-pipéridinyl) propyl] phényl] estra-1,3,5(10)-triène-3,17β-diol.

**5.** Procédé de préparation des composés de formule générale (I) telle que définie à la revendication 1, **caractérisé en ce que** l'on soumet un composé de formule générale (II) :

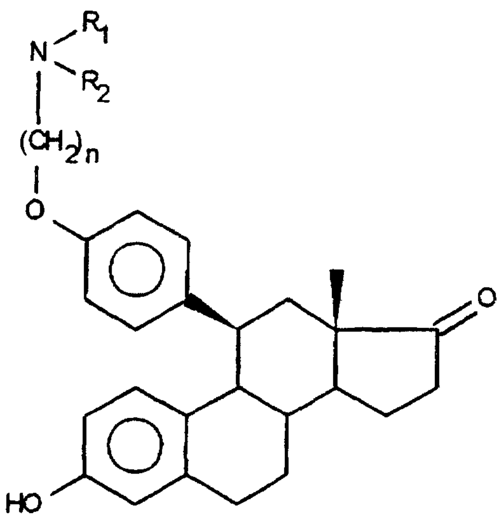

(II)

dans laquelle n, $R_1$ et $R_2$ sont tels que définis à la revendication 1, à l'action d'un composé organométallique dérivé d'un radical alkyle, renfermant de 1 à 4 atomes de carbone afin de former les composés de formule (I) dans laquelle X est un groupement hydroxyle et Y est un groupement alkyle renfermant de 1 à 4 atomes de carbone, composé de formule (I) que l'on soumet le cas échéant à une réaction d'estérification du 17-OH et/ou à une réaction de salification.

**6.** Procédé de préparation des composés de formule générale (I) telle que définie à la revendication 1, avec Y représentant un radical alkyle renfermant de 2 à 4 atomes de carbone, **caractérisé en ce que** l'on soumet un composé de formule générale (III) :

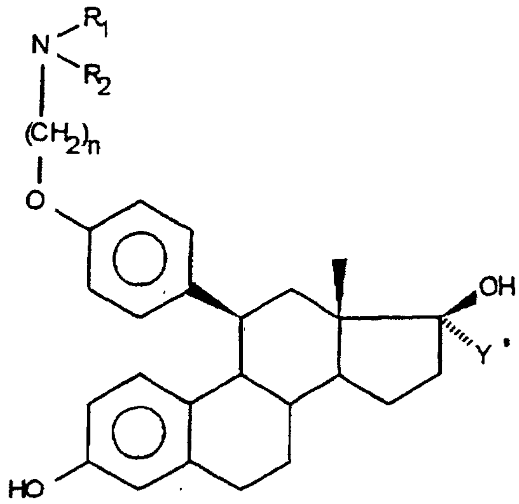

(III)

dans laquelle n, $R_1$ et $R_2$ sont tels que définis précédemment et dans laquelle Y' représente un groupement alkényle ou alkényle renfermant de 2 à 4 atomes de carbone, à l'action de l'agent de réduction de la double liaison ou de la triple liaison, afin d'obtenir les composés de formule (I) dans laquelle X est un groupement hydroxyle et Y est un groupement alkyle renfermant de 2 à 4 atomes de carbone, composé de formule (I) que l'on soumet le cas échéant à une réaction d'estérification du 17-OH et/ou à une réaction de salification.

**7.** A titre de médicaments les composés de formule générale (I) telle que définie à la revendication 1.

**8.** A titre de médicaments les composés de formule générale (I) tels que définis à la revendication 2, 3 ou 4.

**9.** A titre de médicaments destinés au traitement hormonal substitutif de la ménopause ou de la périménopause, en particulier à la prévention ou au traitement de l'ostéoporose, ne présentant que peu ou pas d'activité estrogène au niveau utérin, les composés de formule générale (I) telle que définie à la revendication 1.

**10.** A titre de médicaments destinés au traitement hormonal substitutif de la ménopause ou de la périménopause, en particulier à la prévention ou au traitement de l'ostéoporose des femmes à risque de tumeur mammaires, les composés de formule générale (I) telle que définie à la revendication 1.

**11.** Compositions pharmaceutiques renfermant les médicaments tels que définis à l'une des revendications 7 à 10.

**12.** Médicament tel que défini à la revendication 7 ou 8 pour la prévention ou le traitement de l'ostéropose.

**13.** Médicament tel que défini à la revendication 7 ou 8 pour la protection cardiovasculaire.

**Patentansprüche**

**1.** Verbindungen der allgemeinen Formel (I):

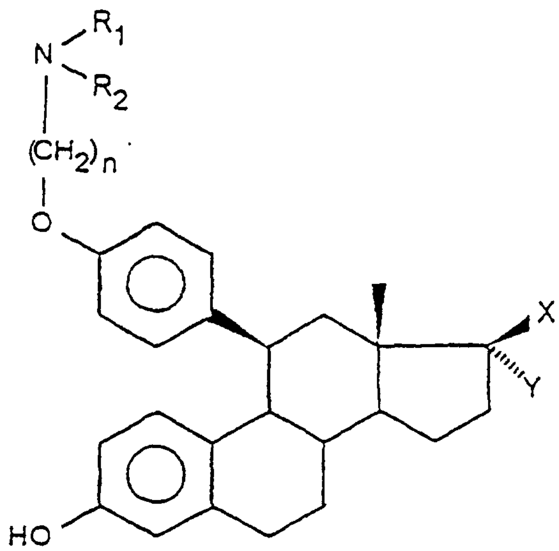

(I)

in der:

n eine ganze Zahl gleich 2 oder 3 ist,
entweder $R_1$ und $R_2$, gleich oder verschieden, ein Wasserstoffatom oder einen Alkylrest mit 1 bis 4 Kohlenstoffatomen darstellen,
oder $R_1$ und $R_2$ zusammen mit dem Stickstoffatom, mit dem sie verbunden sind, einen mono- oder polycyclischen, gesättigten oder ungesättigten, aromatischen oder nicht aromatischen Heterocyclus mit 5 bis 15 Kettengliedern bilden, der gegebenenfalls 1 bis 3 zusätzliche Heteroatome, ausgewählt unter Sauerstoff, Schwefel und Stickstoff, substituiert oder nicht, enthält,
X einen gegebenenfalls veresterten Hydroxyrest darstellt und
Y einen Alkylrest mit 1 bis 4 Kohlenstoffatomen, substituiert oder nicht, darstellt,

sowie deren Additionssalze mit pharmazeutisch annehmbaren Säuren.

**2.** Verbindungen der allgemeinen Formel (I) so wie in Anspruch 1 definiert, in der n gleich 2 ist, sowie deren Additionssalze mit pharmazeutisch annehmbaren Säuren.

**3.** Verbindungen der allgemeinen Formel (1) so wie in Anspruch 1 oder 2 definiert, in der n gleich 2 ist,
entweder $R_1$ und $R_2$, gleich oder verschieden, einen Alkylrest mit 1 bis 4 Kohlenstoffatomen darstellen,
oder $R_1$ und $R_2$ zusammen mit dem Stickstoffatom, mit dem sie verbunden sind, eine Piperidino-, Pyrrolidino- oder 2-Aza-bicyclo(2.2.1)hept-2-yl-Gruppe bilden,
X einen Hydroxyrest darstellt und
Y einen Methyl- oder Ethylrest darstellt,
sowie deren Additionssalze mit pharmazeutisch annehmbaren Säuren.

**4.** Verbindungen der allgemeinen Formel (I) so wie in einem der Ansprüche 1 bis 3 definiert, sowie deren Additionssalze mit pharmazeutisch annehmbaren Säuren, deren Namen folgen:

- 11β-[4-[2-(1-Piperidinyl)ethoxy]phenyl]-19-nor-17α-pregna-1,3,5(10)-trien-3,17β-diol,
- 17α-Methyl-11β-[4-[2-(1-piperidinyl)ethoxy]phenyl]-estra-1,3,5(10)-trien-3,17β-diol,
- 17α-Methyl-11β-[4-[2-(diethylamino)ethoxy]phenyl]-estra-1,3,5(10)-trien-3,17β-diol,
- 17α-Methyl-11β-[4-[2-(1-pyrrolidinyl)ethoxy]phenyl]-estra-1,3,5(10)-trien-3,17β-diol,
- 17α-Methyl-11β-[4-[2-(2-aza-bicyclo(2.2.1)hept-2-yl)ethoxy]phenyl]-estra-1,3,5(10)-trien-3,17β-diol,
- 11β-[4-[2-(2-Aza-bicyclo(2.2.1)hept-2-yl)ethoxy]phenyl]-19-nor-17α-pregna-1,3,5(10)-trien-3,17β-diol,
- 17α-(Trifluormethyl)-11β-[4-[3-(1-piperidinyl)propyl]phenyl]-estra-1,3,5(10)-trien-3,17β-diol.

**5.** Verfahren zur Herstellung von Verbindungen der allgemeinen Formel (I) so wie in Anspruch 1 definiert, **dadurch gekennzeichnet, dass** man eine Verbindung der allgemeinen Formel (II):

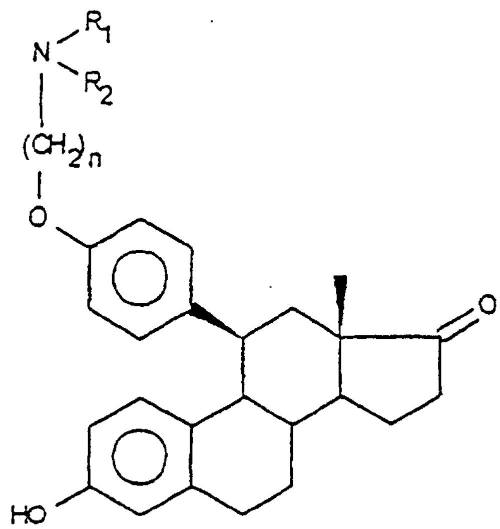

(II)

in der n, $R_1$ und $R_2$ so sind wie in Anspruch 1 definiert, der Wirkung einer organometallischen Verbindung, abgeleitet von einem Alkylrest mit 1 bis 4 Kohlenstoffatomen, unterzieht, um die Verbindungen der Formel (I), in der X eine Hydroxygruppe ist und Y eine Alkylgruppe mit 1 bis 4 Kohlenstoffatomen ist, zu bilden, eine Verbindung der Formel (I), die man gegebenenfalls einer Veresterungsreaktion des 17-OH und/oder einer Salzbildungsreaktion unterzieht.

**6.** Verfahren zur Herstellung von Verbindungen der allgemeinen Formel (I) so wie in Anspruch 1 definiert, wobei Y einen Alkylrest mit 2 bis 4 Kohlenstoffatomen darstellt, **dadurch gekennzeichnet, dass** man eine Verbindung der allgemeinen Formel (III):

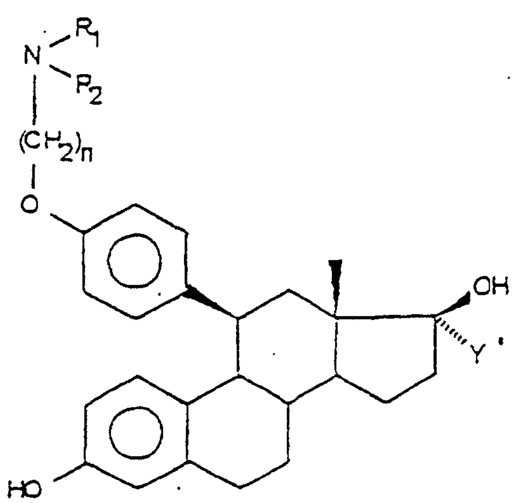

(III)

in der n, $R_1$ und $R_2$ so sind wie vorstehend definiert und in der Y' eine Alkenyl- oder Alkinylgruppe mit 2 bis 4 Kohlenstoffatomen darstellt, der Wirkung eines Mittels zur Reduktion der Doppelbindung oder der Dreifachbindung unterzieht, um die Verbindungen der Formel (I), in der X ein Hydroxygruppe ist und Y eine Alkylgruppe mit 2 bis 4 Kohlenstoffatomen ist, zu erhalten, eine Verbindung der Formel (I), die man gegebenenfalls einer Veresterungsreaktion des 17-OH und/oder einer Salzbildungsreaktion unterzieht.

**7.** Als Medikamente die Verbindungen der allgemeinen Formel (I) so wie in Anspruch 1 definiert.

**8.** Als Medikamente die Verbindungen der allgemeinen Formel (I) so wie in Anspruch 2, 3 oder 4 definiert.

**9.** Als Medikamente, die zur Ersatzhormonbehandlung der Menopause oder der Perimenopause bestimmt sind, insbesondere zur Vorbeugung oder zur Behandlung der Osteoporose, wobei sie nur wenig oder keine Estrogenwirkung auf Gebärmutterebene aufweisen, die Verbindungen der allgemeinen Formel (I) so wie in Anspruch 1 definiert.

**10.** Als Medikamente, die zur Ersatzhormonbehandlung der Menopause oder der Perimenopause bestimmt sind, insbesondere zur Vorbeugung oder zur Behandlung der Osteoporose von Frauen mit Brusttumorrisiko, die Verbindungen der allgemeinen Formel (I) so wie in Anspruch 1 definiert.

**11.** Pharmazeutische Zusammensetzungen, die die Medikamente so wie in einem der Ansprüche 7 bis 10 definiert enthalten.

**12.** Medikament so wie in Anspruch 7 oder 8 definiert zur Verbeugung oder Behandlung von Osteoporose.

**13.** Medikament so wie in Anspruch 7 oder 8 definiert zum Kardiovaskulärschutz.

**Claims**

**1.** The compounds of general formula (I):

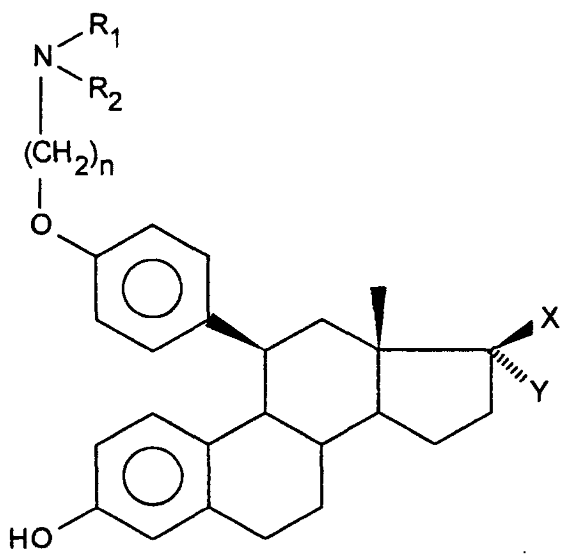

(I)

in which:

n is an integer equal to 2 or 3,
either $R_1$ and $R_2$ identical or different represent a hydrogen atom or an alkyl radical containing 1 to 4 carbon atoms,
or $R_1$ and $R_2$ together with the nitrogen atom to which they are linked form a mono or polyclic, saturated or unsaturated, aromatic or non-aromatic heterocycle with 5 to 15 members, optionally containing 1 to 3 additional heteroatoms chosen from oxygen, sulphur and nitrogen, substituted or non-substituted,
X represents an optionally esterified hydroxyl radical and Y represents an alkyl radical containing 1 to 4 carbon atoms, substituted or non-substituted, as well as their addition salts with pharmaceutically acceptable acids.

**2.** Compounds of general formula (I) as defined in claim 1 in which n is equal to 2, as well as their addition salts with pharmaceutically acceptable acids.

**3.** Compounds of general formula (I) as defined in claim 1 or 2 in which n is equal to 2,
either $R_1$ and $R_2$ identical or different represent an alkyl radical containing 1 to 4 carbon atoms,

or $R_1$ and $R_2$ together with the nitrogen atom to which they are linked form a piperidino, pyrrolidino or 2-azabicyclo (2,2,1)hept-2-yl group,
X represents a hydroxyl radical and Y represents a methyl or ethyl radical, as well as their addition salts with pharmaceutically acceptable acids.

**4.** Compounds of general formula (I) as defined in any one of claims 1 to 3 as well as their addition salts with pharmaceutically acceptable acids the names of which follow:

- 11β-[4-[2-(1-piperidinyl)ethoxy]phenyl)-19-nor-17α-pregna-1,3,5(10)-trien-3,17β-diol,
- 17α-methyl-11β-[4-[2-(1-piperidinyl)ethoxy]phenyl]-estra-1,3,5(10)-trien-3,17β-diol,
- 17α-methyl-11β-[4-[2-(diethylamino)ethoxy]phenyl]-estra-1,3,5(10)-3,17β-diol,
- 17α-methyl-11β-[4-[2-(1-pyrrolidinyl)ethoxy]phenyl]-estra-1,3,5(10)-trien-3,17β-diol,
- 17α-methyl-11β-[4-[2-(2-aza-bicyclo(2,2,1)hept-2-yl) ethoxy]phenyl]-estra-1,3,5(10)-trien-3,17β-diol,
- 11β-[4-[2-(2-aza-bicyclo(2,2,1)hept-2-yl) ethoxy]phenyl]-19-nor-17α-pregna-1,3,5(10)-trien-3,17β-diol,
- 17α-(trifluoromethyl) 11β-[4-[2-(1-piperidinyl)ethoxy] phenyl]-estra-1,3,5(10)-trien-3,17β-diol.

**5.** Process for the preparation of the compounds of general formula (I) as defined in claim 1, **characterized in that** a compound of general formula (II):

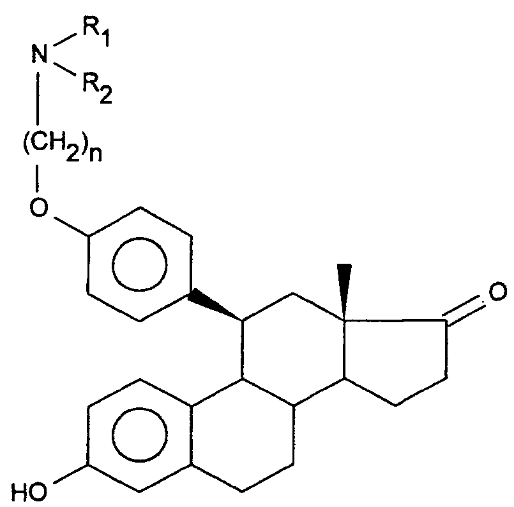

(II)

in which n, $R_1$ and $R_2$ are as defined in claim 1, is subjected to the action of an organometallic compound derivative of an alkyl radical, containing 1 to 4 carbon atoms in order to form the compounds of formula (I) in which X is a hydroxyl group and Y is an alkyl group containing 1 to 4 carbon atoms, which compound of formula (I) is subjected if appropriate to an esterification reaction of the 17-OH and/or to a salification reaction.

**6.** Process for the preparation of the compounds of general formula (I) as defined in claim 1, with Y representing an alkyl radical containing 2 to 4 carbon atoms, **characterized in that** a compound of general formula (III):

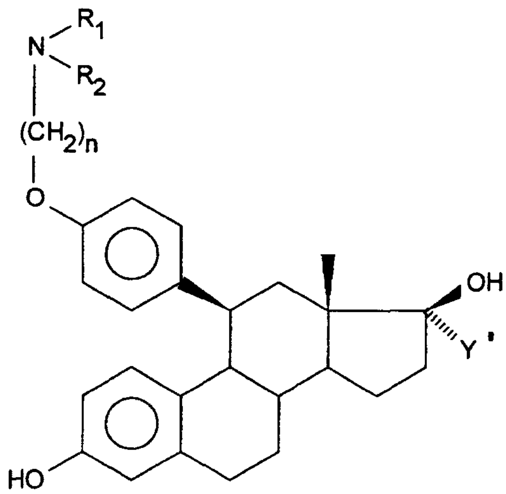

(III)

in which n, $R_1$ and $R_2$ are as defined previously and in which Y' represents an alkenyl or alkynyl group containing 2 to 4 carbon atoms, is subjected to the action of a reducing agent of the double bond or the triple bond, in order to obtain the compounds of formula (I) in which X is a hydroxyl group and Y is an alkyl group containing 2 to 4 carbon atoms, which compound of formula (I) is subjected if appropriate to an esterification reaction of the 17-OH and/or to a salification reaction.

**7.** As medicaments the compounds of general formula (I) as defined in claim 1.

**8.** As medicaments the compounds of general formula (I) as defined in claim 2, 3 or 4.

**9.** As medicaments intended for hormone replacement therapy for the menopause or the perimenopause, in particular for the prevention or treatment of osteoporosis, having little or no estrogenic activity at the level of the uterus, the compounds of general formula (I) as defined in claim 1.

**10.** As medicaments intended for hormone replacement therapy for the menopause or the perimenopause, in particular for the prevention or treatment of osteoporosis in women at risk from breast tumors, the compounds of general formula (I) as defined in claim 1.

**11.** Pharmaceutical compositions containing the medicaments as defined in one of claims 7 to 10.

**12.** Medicament as defined in claim 7 or 8 for the prevention or treatment of osteroposis.

**13.** Medicament as defined in claim 7 or 8 for cardiovascular protection.